# EUROPEAN PATENT APPLICATION

(11) **EP 4 234 664 A1**
(43) Date of publication of application: **30.08.2023**
(21) Application number: 22158412.1
(22) Date of filing: 24.02.2022
(51) Int. Cl.: C11D 1/04, C11D 3/386, C12N 9/96

(54) **COMPOSITION COMPRISING GLUCOLIPIDS AND ENZYMES**

(71) Applicant: Evonik Operations GmbH, 45128 Essen (DE)
(72) Inventor: LIEBIG, Stefan Julian, 40477 Düsseldorf (DE); MÜLLER, Jakob, 45721 Haltern am See (DE); TRAMBITAS, Alexandra, 63755 Alzenau (DE)
(74) Representative: Evonik Patent Association

(57) **Abstract**

The invention relates to compositions comprising at least one enzyme and at least one glucolipid.

## Description

### Field of the invention

The invention relates to compositions comprising at least one enzyme and at least one glucolipid.

### Prior art

Enzymes are used in washing, cleaning and rinsing compositions and, by means of the biocatalytic degradation, contribute to the dissolution of the dirt and thus to the cleaning performance. Prominent examples of enzymes used in cleaning applications are lipases, proteases, amylases and cellulase. The stability of the enzymes in the mostly anionic surfactant systems is still a challenge for the formulator, especially if liquid formulations are to be produced which are storage-stable over a long period of time and should retain their enzymatic activity. In principle, surfactants contribute to the denaturation of proteins and thus of the enzyme structure and thereby cause inactivation of the enzyme activity.

Enzyme producers use protein engineering methods to develop enzymes having increased stability with respect to surfactants. However, this is complex, not successful for all enzymes and may lead to a decreased specific activity due to the altered protein structure.

Alternatively, enzymes may be stabilised by using milder surfactants. For instance, sodium lauryl ether sulphate and/or fatty alcohol ethoxylates are added to the linear alkylbenzene sulphonates used as main surfactant, cf. Kravetz et al. 1985, Lund et al. 2012.

According to US5156773, betaines may also be used to stabilise proteases with respect to anionic surfactants.

DE102007005419 discloses washing or cleaning agent containing surfactant(s) and other customary ingredients of washing or cleaning agents, wherein the agent comprises 0.01 to 5% by weight of one or more enzymes and 0.01 to 20% by weight of a nitrogen-containing surfactant compound.

US5998344 discloses a detergent gel for cleaning of hard surfaces containing: (a) a detergent composition without thickeners having (i) a first surfactant, wherein the first surfactant is a non-ionic glycolipid, and (ii) a second surfactant, wherein the second surfactant is a charged surfactant, wherein the ratio of the first surfactant and the second surfactant is 1:10-10:1; (b) water, wherein the detergent composition is mixed with a sufficient amount of water to induce a phase change from a liquid phase to a gel phase; and (c) one or more enzymes.

WO2017036901 discloses laundry compositions comprising lipases from *Psychromonas ingrahamii.*

WO2012010405 discloses cleaning compositions comprising an effective amount of surfactant system and an enzyme system characterised in that the surfactant system comprises at least 1 wt% (based on the cleaning composition) of a biosurfactant of bacterial origin and at least one enzyme of bacterial origin selected from the group comprising: cellulases, lipases, esterases, peroxidases/oxidases, oxidoreductases, pectases, lyases, mannanases and mixtures thereof. WO2012010406 discloses detergent compositions comprising rhamnolipids and lipase, wherein the weight percent of the rhamnolipid made up by mono-rhamnolipids is more than 50, preferably more than 80, more preferably more than 90 wt%.

WO2012010407 discloses cleaning compositions comprising an effective amount of surfactant system and an enzyme system characterised in that the surfactant system comprises at least 1 wt% (based on the cleaning composition) of a biosurfactant, which is a glycolipid surfactant, comprising at least 20 mol% of glycolipid having both disaccharide and acid moieties and at least one lipase enzyme of bacterial origin.

Biochemistry 2017 56 (32), 4256-4 discloses a study of the interactions between the lipase from *Thermomyces lanuginosus* and the two microbially produced biosurfactants rhamnolipid and sophorolipid.

It is an object of the invention to provide for compositions that stabilize enzymes while having superior cleaning capabilities.

### Description of the invention

It was found that, surprisingly, that enzyme activities can be significantly enhanced by the presence of certain glucolipids, which compared to other sugar based lipids bear no methyl-group within the sugar-residue.

The present invention therefore relates to compositions comprising at least one enzyme and at least one certain glucolipid as describes in claim 1 and their use.

The invention further provides the use of certain glucolipids for the stabilization of an enzymatic activity of an enzyme.

One advantage of the present invention is that the used surfactants are 100% from renewable resources.

Another advantage of the present invention is that the major raw materials for the production of the here presented biosurfactants are sugars or sugars and triglycerides and/or fatty acids.

Another advantage of the composition according to the invention is that it causes a pleasant skin feel.

Another advantage of the present invention is that the compositions according to the instant invention have an improved colour stability.

One advantage of the present invention is that the compositions according to the instant invention have a positive odour profile.

A further advantage is that the compositions according to the instant invention have outstanding cleaning properties of surfaces.

Another advantage is that the compositions according to the instant invention has excellent foaming properties.

A further advantage is that the compositions according to the instant invention is their low irritation for human skin.

Another advantage is that the compositions according to the instant invention is that they leave behind a smooth and soft skin feel after washing.

Another advantage of the compositions according to the instant invention is their outstanding microbial stability.

Another advantage is that the compositions according to the instant invention have an improved ability to dissolve oils and fats.

Another advantage of a composition according to the invention is its outstanding mildness. Another advantage of the present invention is that the amount of protease inhibitor can be reduced or that the protease inhibitor can be omitted.

Another advantage of the present invention is that the amount of any enzyme inhibitor can be reduced or that the usage of an enzyme inhibitor can be omitted.

Another advantage of the present invention is an increased enzyme stability during application in laundry and the resulting increase of cleaning effectiveness.

Another advantage of the present invention is that it allows the use a broader spectrum of proteases in detergent formulation, which is usually limited due to their insufficient stability. Another advantage of the present invention is, that less or no complexing agent (builders) needs to be used in laundry application to reach a high washing performance even in water with high water hardness.

The instant invention thus provides a composition comprising
A) at least one enzyme, and
B) at least one glucolipid of general formula (I) or a salt thereof, 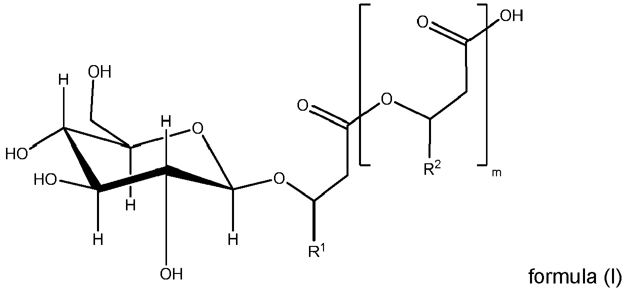
where
m = 1 or 0,
R¹ and R² = independently of one another identical or different organic radical having 2 to 24 carbon atoms, in particular optionally branched, optionally substituted, in particular hydroxy-substituted, optionally unsaturated, in particular optionally mono-, di- or triunsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12.

It is obvious for a person skilled in the art, that the glucolipids of general formula (I) encompass different stereoisomers.

Distinct glucolipids are abbreviated according to the following nomenclature:
"GL-CXCY" is understood as meaning glucolipids of the general formula (I) in which one of the radicals R¹ and R² = (CH₂)ₒ-CH₃ where o = X-4 and the remaining radical R¹ or R² = (CH₂)ₒ-CH₃ where o = Y-4.

The nomenclature used thus does not differentiate between "CXCY" and "CYCX".

If one of the aforementioned indices X and/or Y is provided with ":Z", then this means that the respective radical R¹ and/or R² = an unbranched, unsubstituted hydrocarbon radical with X-3 or Y-3 carbon atoms having Z double bonds.

Methods for production of glucolipids as used in the context of the instant invention can be carried out as described in WO2019154970.

In connection with the present invention, the "pH" is defined as the value which is measured for a corresponding substance at 25°C after stirring for five minutes using a pH electrode calibrated in accordance with ISO 4319 (1977).

In connection with the present invention, the term "aqueous medium" is understood as meaning a composition which comprises at least 5% by weight of water, based on the total composition under consideration.

The term "total dry mass" in the context of the present invention is understood to mean the portion of the mixture composition according to the invention which remains - naturally in addition to water - after the composition according to the invention has been freed of the components which are liquid at 25°C and 1 bar.

In the context of the present invention, the terms "surfactant" is understood to mean organic substances having interface-active properties that have the ability to reduce the surface tension of water at 20°C and at a concentration of 0.5% by weight based on the overall composition to below 45 mN/m. Surface tension is determined by the DuNoüy ring method at 20°C.

Within the context of the present invention, "biosurfactants" are understood as meaning all glycolipids produced by fermentation. The term "biosurfactant" also covers glycolipids that are chemically or enzymatically modified after fermentation, as long as structurally a glycolipid remains.

Raw materials for producing the biosurfactants that can be used are carbohydrates, in particular sugars such as e.g. glucose and/or lipophilic carbon sources such as fats, oils, partial glycerides, fatty acids, fatty alcohols, long-chain saturated or unsaturated hydrocarbons.

Unless stated otherwise, all percentages (%) given are percentages by mass.

A preferred composition according to the invention comprises glucolipids of the general formula (I) or a salt thereof, where m=1 and R¹ and R² = independently of one another identical or different organic radical having 2 to 24 carbon atoms,
characterized in that the composition comprises
at least 51% by weight to preferably 98% by weight, preferably 60% by weight to 95% by weight, more preferably 70% by weight to 90% by weight, particularly preferably 75% by weight to 85% by weight, glucolipids GL-C10C10 of the general formula (I) with m=1 and R¹ and R² = (CH₂)₆-CH₃, where the percentages by weight refer to the sum of all of the glucolipids of the general formula (I) present.

A preferred composition according to the invention is characterized in that the pH of the mixture composition at 25°C is from 3.5 to 9, preferably from 5 to 7 and particularly preferably from 5.6 to 6.6.

The glucolipids present in the composition according to the invention are present at least partially as salts on account of the given pH.

In preferred mixture compositions according to the invention the cations of the glucolipid salts present are selected from the group comprising, preferably consisting of, Li⁺, Na⁺, K⁺, Mg²⁺, Ca²⁺, Al³⁺, NH₄⁺, primary ammonium ions, secondary ammonium ions, tertiary ammonium ions and quaternary ammonium ions.

Exemplary representatives of suitable ammonium ions are tetramethylammonium, tetraethylammonium , tetrapropylammonium, tetrabutylammonium and [(2-hydroxyethyl)trimethylammonium] (choline) and also the cations of 2-aminoethanol (ethanolamine, MEA), diethanolamine (DEA), 2,2',2"-nitrilotriethanol (triethanolamine, TEA), 1-aminopropan-2-ol (monoisopropanolamine), ethylenediamine, diethylenetriamine, triethylenetetramine, tetraethylenepentamine, 1,4-diethylenediamine (piperazine), aminoethylpiperazine and aminoethylethanolamine.

The mixtures of the abovementioned cations may also be present as cations of the glucolipid salts present according to the invention.

Particularly preferred cations are selected from the group comprising, preferably consisting of, Na⁺, K⁺, NH₄⁺ and the triethanolammonium cation.

It may be advantageous and is therefore preferred if the composition according to the invention comprises
1% by weight to 30% by weight, preferably 5% by weight to 25% by weight, particularly preferably 10% by weight to 20% by weight, of GL-C8C10,
where the percentages by weight refer to the sum of all of the glucolipids of the general formula (I) present.

A preferred mixture composition according to the invention is characterized in that the composition comprises
0.5% by weight to 20% by weight, preferably 3% by weight to 17% by weight, particularly preferably 5% by weight to 15% by weight, of GL-C10C12:1,
where the percentages by weight refer to the sum of all of the glucolipids of the general formula (I) present.

A further preferred mixture composition according to the invention is characterized in that the composition comprises
0.5% by weight to 20% by weight, preferably 2% by weight to 15% by weight, particularly preferably 3% by weight to 12% by weight, of GL-C10C12
where the percentages by weight refer to the sum of all of the glucolipids of the general formula (I) present.

A particularly preferred mixture composition according to the invention is characterized in that the composition comprises
1% by weight to 30% by weight, preferably 5% by weight to 25% by weight, particularly preferably 10% by weight to 20% by weight, of GL-C8C10,
0.5% by weight to 20% by weight, preferably 3% by weight to 17% by weight, particularly preferably 5% by weight to 15% by weight, of GL-C10C12:1,
0.5% by weight to 20% by weight, preferably 2% by weight to 15% by weight, particularly preferably 3% by weight to 12% by weight, of GL-C10C12
where the percentages by weight refer to the sum of all of the glucolipids of the general formula (I) present.

A very particularly preferred composition according to the invention is characterized in that the mixture composition comprises
10% by weight to 20% by weight, of GL-C8C10,
5% by weight to 15% by weight, of GL-C10C12:1,
3% by weight to 12% by weight, of GL-C10C12
where the percentages by weight refer to the sum of all of the glucolipids of the general formula (I) present.

Over and above this, it is preferred if the composition according to the invention comprises glucolipids of the formula GL-CX in only small amounts. In particular, the composition according to the invention comprises preferably
0% by weight to 5% by weight, preferably 0,01% by weight to 4% by weight, particularly preferably 0,1% by weight to 3% by weight, of GL-C10,
where the percentages by weight refer to the sum of all of the glucolipids of the general formula (I) present.

Enzymes are useful additives in laundry compositions. The enzymes preferably comprised in the composition according to the instant invention are selected from the group consisting of protease, amylase, cellulase, mannanase, lipase, cutinase, pectate lyase, peroxidase, oxidase and laccase with protease, amylase, lipase and mannanase being especially preferred and lipase being the most preferred.

Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium , e.g., the fungal cellulases produced from Humicola insolens, Myceliophthora thermophila and Fusarium oxysporum disclosed in U.S. Pat. Nos. 4,435,307, 5,648,263, 5,691,178, 5,776,757 and WO 89/09259.

Especially suitable cellulases are the alkaline or neutral cellulases having color care benefits. Examples of such cellulases are cellulases described in EP 0 495 257, EP 0 531372, WO 96/11262, WO 96/29397, WO 98/08940. Other examples are cellulase variants such as those described in WO 94/07998, EP 0 531315, U.S. Pat. Nos. 5,457,046, 5,686,593, 5,763,254, WO 95/24471, WO 98/12307 and PCT/DK98/00299.

Example of cellulases exhibiting endo-beta-1,4-glucanase activity (EC 3.2.1.4) are those having described in WO02/099091.

Other examples of cellulases include the family 45 cellulases described in WO96/29397, and especially variants thereof having substitution, insertion and/or deletion at one or more of the positions corresponding to the following positions in SEQ ID NO: 8 of WO 02/099091: 2, 4, 7, 8, 10, 13, 15, 19, 20, 21, 25, 26, 29, 32, 33, 34, 35, 37, 40, 42, 42a, 43, 44, 48, 53, 54, 55, 58, 59, 63, 64, 65, 66, 67, 70, 72, 76, 79, 80, 82, 84, 86, 88, 90, 91, 93, 95, 95d, 95h, 95j, 97, 100, 101, 102, 103, 113, 114, 117, 119, 121, 133, 136, 137, 138, 139, 140a, 141, 143a, 145, 146, 147, 150e, 150j, 151, 152, 153, 154, 155, 156, 157, 158, 159, 160c, 160e, 160k, 161, 162, 164, 165, 168, 170, 171, 172, 173, 175, 176, 178, 181, 183, 184, 185, 186, 188, 191, 192, 195, 196, 200, and/or 20, preferably selected among P19A, G20K, Q44K, N48E, Q119H or Q146 R.

Commercially available cellulases include Celluzyme^{™}, and Carezyme^{™} (Novozymes A/S), Clazinase^{™}, and Puradax HA^{™} (Genencor International Inc.), and KAC-500(B)^{™} (Kao Corporation).

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from Bacillus such as Bacillus lentus, B. alkalophilus, B. subtilis, B. amyloliquefaciens, Bacillus pumilus and Bacillus gibsonii described in; U.S. Pat. No. 7,262,042 and WO09/021867, and subtilisin lentus , subtilisin Novo, subtilisin Carlsberg, Bacillus licheniformis , subtilisin BPN', subtilisin 309, subtilisin 147 and subtilisin 168 described in WO89/06279 and protease PD138 described in (WO93/18140). Other useful proteases may be those described in WO92/175177, WO1/016285, WO02/026024 and WO02/016547. Examples of trypsin-like proteases are trypsin (e.g. of porcine or bovine origin) and the Fusarium protease described in WO89/06270, WO94/25583 and WO05/040372, and the chymotrypsin proteases derived from Cellumonas described in WO05/052161 and WO05/052146.

A further preferred protease is the alkaline protease from Bacillus lentus DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from Bacillus amyloliquefaciens.

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V2051, L217D, N218D, M222S, A232V, K235L, Q236H, Q245R, N252K, T274A (using BPN' numbering).

Suitable commercially available protease enzymes include those sold under the trade names Alcalase^{®}, Duralase^{™}, Durazym^{™}, Relase^{®}, Relase^{®} Ultra, Savinase^{®}, Savinase^{®} Ultra, Primase, Polarzyme^{®}, Kannase^{®}, Liquanase^{®}, Liquanase^{®} Ultra, Liquanase^{®} 2.5 L, Ovozyme^{®}, Coronase, Coronase^{®} Ultra, Neutrase^{®}, Everlase^{®} and Esperase^{®} (Novozymes A/S), those sold under the tradename Maxatase^{®}, Maxacal^{®}, Maxapem^{®}, Purafect^{®}, Purafect Prime^{®}, Eraser^{®}, Purafect MA^{®}, Purafect Ox^{®}, Purafect OxP^{®}, Puramax^{®}, Properase^{®}, Ultimase^{®}, FN2^{®}, FN3^{®}, FN4^{®}, Excellase^{®}, , Opticlean^{®} and Optimase^{®} (Danisco/DuPont), Axapem^{™} (Gist-Brocases N.V.), BLAP (sequence shown in FIG. 29 of U.S. Pat. No. 5,352,604) and variants hereof (Henkel AG) and KAP (Bacillus alkalophilus subtilisin) from Kao.

A protease preferably comprised in the composition according to the instant invention is Liquanase^{®} 2.5 L.

Suitable lipases and cutinases include those of bacterial or fungal origin. Chemically modified or protein engineered mutant enzymes are included. Examples include lipase from Thermomyces , e.g. from T. lanuginosus (previously named Humicola lanuginosa ) as described in EP258068 and EP305216, cutinase from Humicola , e.g. H. insolens (WO96/13580), lipase from strains
of Pseudomonas (some of these now renamed to Burkholderia), e.g. P. alcaligenes or P. pseudoalcaligenes (EP218272), P. cepacia (EP331376), P. sp. strain SD705 (WO95/06720 & WO96/27002), P. wisconsinensis (WO96/12012), GDSL-type Streptomyces lipases (WO10/065455), cutinase from Magnaporthe grisea (WO10/107560), cutinase from Pseudomonas mendocina (U.S. Pat. No. 5,389,536), lipase from Thermobifida fusca (WO11/084412), Geobacillus stearothermophilus lipase (WO11/084417), lipase from Bacillus subtilis (WO11/084599), and lipase from Streptomyces griseus (WO11/150157) and S. pristinaespiralis (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

A lipase preferably comprised in the composition according to the instant invention are lipase isolated from Stachybotrys, especially the lipase with Seq ID No. 1 in CN104031899 and variants thereof.

Preferred commercial lipase products include Lipolase^{™}, Lipex^{™}; Lipolex^{™}, Lipex^{™} 100 L Evity and Lipoclean^{™} (Novozymes A/S), Lumafast (originally from Genencor) and Lipomax (originally from Gist-Brocades).

A lipase preferably comprised in the composition according to the instant invention is Lipex^{™} 100 L Evity.

Still other examples are lipases sometimes referred to as acyltransferases or perhydrolases, e.g. acyltransferases with homology to Candida antarctica lipase A (WO10/111143), acyltransferase from Mycobacterium smegmatis (WO05/56782), perhydrolases from the CE 7 family (WO09/67279), and variants of the M. smegmatis perhydrolase in particular the S54V variant used in the commercial product Gentle Power Bleach from Huntsman Textile Effects Pte Ltd (WO10/100028).

Suitable amylases which can be used herein may be an alpha-amylase or a glucoamylase and may be of bacterial or fungal origin. Chemically modified or protein engineered mutants are included. Amylases include, for example, alpha-amylases obtained from Bacillus , e.g., a special strain of Bacillus licheniformis , described in more detail in GB 1,296,839.

Suitable amylases include amylases having SEQ ID NO: 3 in WO 95/10603 or variants having about 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having about 90% sequence identity thereto. Preferred variants are those having a deletion in positions 181 and 182 and a substitution in position 193.

Other amylases which are suitable are hybrid alpha-amylase comprising residues 1-33 of the alpha-amylase derived from B. amyloliquefaciens shown in SEQ ID NO: 6 of WO 2006/066594 and residues 36-483 of the B. licheniformis alpha-amylase shown in SEQ ID NO: 4 of WO 2006/066594 or variants having about 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from B. amyloliquefaciens shown in SEQ ID NO: 6 of WO 2006/066594 and residues 36-483 of SEQ ID NO: 4 of WO2006/066594 are those having the substitutions:
M197T;H156Y+A181T+N190F+A209V+Q264S;
orG48A+T49I+G107A+H156Y+A181T+N190F+I201F+A209V+Q264S.

Further amylases which are suitable are amylases having SEQ ID NO: 6 in WO 99/019467 or variants thereof having about 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184. Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having about 90% sequence identity to SEQ ID NO: 2 of WO 08/153815 or about 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264. Further suitable amylases are amylases having SEQ ID NO: 2 of WO 09/061380 or variants having about 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;N128C+K178L+T182G+F202Y+Y305R+D319T+G475K;S 125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least about 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Amplify^{™} Prime 100 L, Duramyl^{™}, Termamyl^{™}, Fungamyl^{™}, Stainzyme^{™}, Stainzyme Plus^{™}, Natalase^{™}, Liquozyme X and BAN^{™} (from Novozymes A/S), and Rapidase^{™}, Purastar^{™}/Effectenz^{™}, Powerase and Preferenz S100 (from Genencor International Inc./DuPont).

Amplify^{™} Prime 100 L is preferably comprised in the composition according to the instant invention

Suitable peroxidases/oxidases include those of plant, bacterial or fungal origin. Chemically modified or protein engineered mutants are included. Examples of useful peroxidases include peroxidases from Coprinus , e.g., from C. cinereus , and variants thereof as those described in WO 93/24618, WO 95/10602, and WO 98/15257.

Commercially available peroxidases include Guardzyme^{™} (Novozymes A/S). The detergent enzyme(s) may be included in a detergent composition by adding separate additives containing one or more enzymes, or by adding a combined additive comprising all of these enzymes. A detergent additive as contemplated herein, i.e., a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, e.g., as disclosed in U.S. Pat. Nos. 4,106,991 and 4,661,452 and may optionally be coated by methods known in the art. Examples of waxy coating materials are poly(ethylene oxide) products (polyethyleneglycol, PEG) with mean molar weights of from about 1000 to about 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

Mannanases which are particularly preferred according to the invention are mannanases which are sold, for example, under the trade names Mannaway^{®} by the company Novozymes or Purabrite^{®} by the company Genencor. A mannanase preferably comprised in the composition according to the instant invention is Mannaway^{®} 4.0 L.

A preferred composition according to the instant invention further comprises C) at least one builder.

Any builder known in the art for use in laundry detergents may be utilized, these can be for example mineral, polymeric and organic builders.

The builder may particularly be a chelating agent that forms water-soluble complexes with calcium and magnesium. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

Homopolymers of polyacrylates or copolymers thereof, such as poly(acrylic acid) (PAA) or copoly(acrylic acid/maleic acid) (PAA/PMA). Further non-limiting examples include polyaspartic acids and polyglutamic acids and their salts, citrates, ascorbic acid chelators such as aminocarboxylates, aminopolycarboxylates, like N,N-dicarboxymethyl glutamic acid and methylglycine N,N-diacetic acid, and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), glutamic acid-N,N-diacetic acid (GLDA), 1-hydroxyethane-1,1-diphosphonic acid (HEDP), ethylenediaminetetra-(methylenephosphonic acid) (EDTMPA), diethylenetriaminepentakis(methylenephosphonic acid) (DTPMPA or DTMPA), N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl)-aspartic acid (SMAS), N-(2-sulfoethyl)-aspartic acid (SEAS), N-(2-sulfomethyl)-glutamic acid (SMGL), N-(2-sulfoethyl)-glutamic acid (SEGL), N-methyliminodiacetic acid (MIDA), α-alanine-N,N-diacetic acid (a-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA), N-(2-hydroxyethyl)-ethylidenediamine-N,N',N'-triacetate (HEDTA), diethanolglycine (DEG), diethylenetriamine penta(methylenephosphonic acid) (DTPMP), aminotris(methylenephosphonic acid) (ATMP), and combinations and salts thereof.

Preferred builder comprised in the composition according to the instant invention are selected from the group of aminopolycarboxylates, like N,N-dicarboxymethyl glutamic acid and methylglycine N,N-diacetic acid, citrates, polyaspartic acids and polyglutamic acids and their salts.

A preferred composition according to the instant invention is characterized in that it comprises
A) in an amount of from 0.2 wt.-% to 10 wt.-%, preferably from 1.0 wt.-% to 8.0 wt.-%, more preferably from 1.5 wt.-% to 6.0 wt.-%, and as may be the case
B) in an amount of from 1.0 wt.-% to 60 wt.-%, preferably from 3.0 wt.-% to 40 wt.-%, more preferably from 6.0 wt.-% to 35 wt.-%, even more preferably from 10.0 wt.-% to 30 wt.-%, and as may be the case,
C) in an amount of from 1.0 wt.-% to 30 wt.-%, preferably from 2.0 wt.-% to 20 wt.-%, more preferably from 3.0 wt.-% to 10 wt.-%,
where the percentages by weight refer to the total composition.

A preferred composition according to the instant invention is characterized in that it comprises at least one further surfactant, preferably selected from the group of non-glucolipid biosurfactants, anionic, cationic, non-ionic, semi-polar and zwitterionic surfactants.

The optionally comprised surfactant is preferably comprised at a maximum of 10 wt.-% of all surfactants contained in the total composition.

Non-limiting examples of non-glucolipid biosurfactants are rhamnolipids and sophorolipids.

Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), polyglycerol esters, glaycerol esters, propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

Non-limiting examples of semipolar surfactants include amine oxides (AO) such as alkyldimethylamineoxide, N-(coco alkyl)-N,N-dimethylamine oxide and N-(tallow-alkyl)-N,N-bis(2-hydroxyethyl)amine oxide, fatty acid alkanolamides and ethoxylated fatty acid alkanolamides, and combinations thereof.

Non-limiting examples of zwitterionic surfactants include betaine, alkyldimethylbetaine, sulfobetaine, and combinations thereof.

The composition according to the instant invention can further comprise one or more auxiliary agents selected from the group consisting of
bleaching systems, hydrotropes, polymers, anti-redeposition aids, fiber protection agents, soil release agents, dye transfer inhibitors, fabric hueing agents, blueing dyes, enzyme stabilizing agents like boric acid, viscosity modifiers, pH-regulators and salts like NaCl and Na₂SO₄.

The composition as contemplated herein preferably is a detergent composition. It may be in any convenient form, e.g., a bar, a homogenous tablet, a tablet having two or more layers, a pouch having one or more compartments, a regular or compact powder, a granule, a paste, a gel, or a regular, compact or concentrated liquid. There are a number of detergent formulation forms such as layers (same or different phases), pouches, as well as forms for machine dosing unit.

Pouches can be configured as single or multicompartments. It can be of any form, shape and material which is suitable for hold the composition, e.g. without allowing the release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxyprpyl methyl cellulose (HPMC). Preferably the level of polymer in the film for example PVA is at least about 60%. Preferred average molecular weight will typically be from about 20,000 to about 150,000. Films can also be of blend compositions comprising hydrolytically degradable and water soluble polymer blends such as polyactide and polyvinyl alcohol (known under the Trade reference M8630 as sold by Chris Craft In. Prod. Of Gary, Ind., US) plus plasticisers like glycerol, ethylene glycerol, Propylene glycol, sorbitol and mixtures thereof. The pouches can comprise a solid laundry detergent composition or part components and/or a liquid cleaning composition or part components separated by the water-soluble film. The compartment for liquid components can be different in composition than compartments containing solids, see for example US20090011970.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

The detergent composition according to the instant invention can be in form of a laundry soap bar and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The detergent composition according to the instant invention can be formulated as a granular detergent as described in WO09/092699, EP1705241, EP1382668, WO07/001262, U.S. Pat. No. 6,472,364, WO04/074419 or WO09/102854.

The detergent composition according to the instant invention preferably is in the form of a liquid or gel detergent. This may be aqueous, typically containing at least 20% by weight water, with the percentages referring to the total composition. Other types of liquids, including without limitation, alkanols, amines, diols, ethers and polyols may be included in an aqueous liquid or gel. An aqueous liquid or gel detergent composition may contain from 0-30 wt.-% organic solvent, with the percentages referring to the total composition. A liquid or gel detergent may be non-aqueous.

A preferred composition according to the invention is characterized in that the pH of the composition at 25°C is from 3.0 to 9, preferably from 4.0 to 7 and particularly preferably from 5.0 to 6.6.

The instant invention further provides the use of a composition according to the instant invention for cleaning a surface of an article, preferably a textile or a fabric.

Preferably the use according to the instant invention is characterized in that the surface of the article is cleaned from fat and/or oil, preferably from solid fatty stains.

The instant invention further provides the use of a glucolipid of general formula (I) for the stabilization and/or elevation of an enzymatic activity of an enzyme, preferably selected from the group consisting of protease, amylase, cellulase, mannanase, lipase, cutinase, pectate lyase, peroxidase, oxidase and laccase with protease, amylase, lipase and mannanase being especially preferred and lipase being the most preferred.

In the uses according to the instant invention the preferred compositions according to the instant invention are preferably used; the same is true for the preferred glucolipids of component A).

The examples adduced hereinafter describe the present invention by way of example, without any intention that the invention, the scope of application of which is apparent from the entirety of the description and the claims, be restricted to the embodiments specified in the examples.

### Examples:

### Example 1: Comparison of the storage stability of a lipase in glucolipids and rhamnolipids

The stabilizing effect of glucolipids and rhamnolipids on lipases is shown. The surfactant mixtures were prepared in a TRIS buffer (0.1 mol/L, pH8.5).

Lipase from *Thermomyces lanuginosus,* commercially available as Lipex^{®} Evity 100L, was used as a lipase.

### Preparation of Glucolipids

Glucolipids were produced according to example 2 of WO2019154970 via fermentation.

Cells were separated by centrifugation at 10.000 g for 20 minutes. The fermentation broth was separated as the supernatant and adjusted to pH 3.1 by addition of concentrated H₂SO₄.

After a second centrifugation at 5.000 g for 20 minutes the aqueous upper phase was separated off and the remaining lower phase was a concentrate which was diluted to 50% for a stock solution.

### Preparation of Rhamnolipid A

Rhamnolipid A was prepared as described in EP3023431 and analysed.

The proportion of rhamnolipids and salts thereof was > 90% by weight based on the dry mass. The relative proportions of the various rhamnolipid congeners in per cent by weight of the sum total of all rhamnolipids are given in the following table. Here, the ratios refer to the acid form which is quantified in the HPLC analysis.

**Table 1: Composition of the Rhamnolipid A used. Data in % by weight of the respective congener (as acid form) based on the sum total of all rhamnolipids (as acid form).**

| | |
|---|---|
| *diRL-C8C10* | *15.8* |
| *diRL-C10C10* | *66.4* |
| *diRL-C10C12:1* | *6.4* |
| *diRL-C10C12* | *6.2* |
| *monoRL-C10C10* | *2.4* |
| *other rhamnolipids* | *2.8* |

The Rhamnolipid A was diluted with water to give a 50 % stock solution of rhamnolipid A.

### Preparation of Rhamnolipid B

The stock solution of Rhamnolipid A was diluted to 1% with water. Two liters of this stock solution were heated to 50°C. A thermostable rhamnosidase (200 units, ThermoActiveTM Rhamnosidase A, Prokazyme) was added under constant stirring and the mixture was allowed to react over night. After 20 h a sample was analysed via HPLC. The di-rhamnolipid was completely transferred into mono-rhamnolipid and rhamnose. The enzyme was inactivated at 80°C for 1h. The sample was freeze-dried. By adding water, the sample was diluted to 50 wt% mono-rhamnolipid content. Two parts of this stock solution were mixed with 3 parts of stock solution Rhamnolipid A to give Rhamnolipid B.

### Preparation of rhamnolipid C

Rhamnolipid C is a 1:1 mixture of the 50%-solutions of mono-rhamnolipids and Rhamnolipid A from above.

The samples 1 to 8 were prepared in 2 mL sample tubes.

| **Sample no.** | **description** |
|---|---|
| 1 | 10% glucolipid solution + 0.2% lipase |
| 2 | 10% rhamnolipid B solution + 0.2% lipase |
| 3 | 10% rhamnolipid C solution + 0.2% lipase |
| 4 | 5% glucolipid solution + 0.2% lipase |
| 5 | 5% rhamnolipid A solution + 0.2% lipase |
| 6 | 2.5% glucolipid solution + 0.2% lipase |
| 7 | 2.5% rhamnolipid A solution + 0.2% lipase |

Samples were taken from the compositions and were diluted 100-fold with 0.1 M TRIS buffer. 100 µL of the diluted samples were pipetted into a 96-well Microplate. A 4-Nitrophenyloctanoate (Sigma-Aldrich) stock solution was prepared immediately before the measurements were started by mixing 325 µL with 975 µL TRIS buffer. 20 µL of the stock solution were added to the 100 µL of the samples. The enzyme activity was measured from the hydrolysed substrate 4-Nitrophenyloctanoate at 24°C in a microplate reader (Tecan, Infinite^{®} M200 Pro) at 410 nm. The relative activity was calculated from the final activity after 60 minutes and related to the enzyme activity of the pure lipase which was stored in a refrigerator. The measurements were repeated after 1,2 or 1,2,3 and 4 weeks.

| | | **concentration** | **weeks** | | | |
|---|---|---|---|---|---|---|
| | | | **1** | **2** | **3** | **4** |
| 1 | Glucolipid | 10% | 114% | 103% | 95% | 95% |
| 2 | Rhamnolipid B | 10% | 97% | 101% | 55% | 29% |
| 3 | Rhamnolipid C | 10% | 97% | 98% | 83% | 51% |

| | | **concentration** | **weeks** | | | |
|---|---|---|---|---|---|---|
| | | | **1** | **2** | **3** | **4** |
| 4 | Glucolipid | 5% | 111% | 100% | 94% | 57% |
| 5 | Rhamnolipid A | 5% | 110% | 100% | 93% | 40% |

| | | **concentration** | **weeks** | |
|---|---|---|---|---|
| | | | **1** | **2** |
| 6 | Glucolipid | 2.5% | 110% | 97% |
| 7 | Rhamnolipid A | 2.5% | 110% | 70% |

### Formulation examples:

### Further Liquid detergents:

| Ingredient | |
|---|---|
| Na soap from palm kernel oil | 2.0 |
| Sodium Citrate | 6.0 |
| GLUCOLIPIDS | 15 |
| Poly L-Aspartic acid Sodium salt | 1.0 |
| Phenoxyethanol | 0.45 |
| Protease | 1.0 |
| Amylase | 0.4 |
| Lipase | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.0 |

### Example Formulation 1

| Ingredient | |
|---|---|
| Na soap from palm kernel oil | 2.0 |
| Sodium Citrate | 6.0 |
| GLUCOLIPIDS | 20 |
| Poly-L-glutamic acid sodium salt | 1.0 |
| Phenoxyethanol | 0.45 |
| Protease | 1.2 |
| Amylase | 0.4 |
| Lipase | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.0 |

### Example Formulation 2

| Ingredient | |
|---|---|
| Na soap from palm kernel oil | 2.0 |
| Sodium Citrate | 6.0 |
| GLUCOLIPIDS | 20 |
| Poly-L-glutamic acid sodium salt | 1.0 |
| Carboxy methyl inulin | 1.0 |
| Phenoxyethanol | 0.45 |
| Protease | 1.2 |
| Amylase | 0.4 |
| Lipase | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.0 |

### Example Formulation 3

| Ingredient | |
|---|---|
| Na soap from palm kernel oil | 2.0 |
| Sodium Citrate | 6.0 |
| GLUCOLIPIDS | 20 |
| Poly-L-glutamic acid sodium salt | 1.0 |
| Carboxy methyl inulin | 1.0 |
| Phenoxyethanol | 0.45 |
| Protease | 1.0 |
| Amylase | 0.4 |
| Lipase from Stachybotrys chlorohalonata | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.0 |

### Example Formulation 4

| Ingredient | |
|---|---|
| Na soap from palm kernel oil | 2.0 |
| Sodium Citrate | 6.0 |
| GLUCOLIPIDS | 20 |
| Carboxy methyl inulin | 3 |
| Phenoxyethanol | 0.45 |
| Protease | 1.0 |
| Amylase | 0.4 |
| Lipase from Stachybotrys chlorohalonata | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.0 |

### Example Formulation 5

| Ingredient | |
|---|---|
| Na soap from palm kernel oil | 2.0 |
| Sodium Citrate | 6.0 |
| MGDA | 1 |
| GLUCOLIPIDS | 20 |
| Poly-L-glutamic acid sodium salt | 1.0 |
| Carboxy methyl cellulose | 1.0 |
| Phenoxyethanol | 0.45 |
| Protease | 1.0 |
| Amylase | 0.4 |
| Lipase from Stachybotrys chlorohalonata | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.3 |

### Example Formulation 6

| Ingredient | |
|---|---|
| Na soap from palm kernel oil | 2.0 |
| Sodium Citrate | 6.0 |
| MGDA | 1 |
| GLUCOLIPIDS | 20 |
| Carboxy methyl cellulose | 3 |
| Phenoxyethanol | 0.45 |
| Protease | 1.0 |
| Amylase | 0.4 |
| Lipase from Stachybotrys chlorohalonata | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.3 |

### Example Formulation 7

| Ingredient | |
|---|---|
| Na soap from palm kernel oil | 2.0 |
| Sodium Citrate | 6.0 |
| GLUCOLIPIDS | 20 |
| Polyitaconic acid | 3 |
| Phenoxyethanol | 0.45 |
| Protease | 1.0 |
| Amylase | 0.4 |
| Lipase from Stachybotrys chlorohalonata | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.3 |

### Example Formulation 8

| Ingredient | |
|---|---|
| Na soap from palm kernel oil | 2.0 |
| Sodium Citrate | 6.0 |
| MGDA | 1 |
| Sodium lauryl ether sulfate (SLES) | 5 |
| GLUCOLIPIDS | 15 |
| Phenoxyethanol | 0.45 |
| Medley^{®} Brilliant 300 L | 2 |
| | Add water to 100 % |
| pH | 8.3 |

### Example Formulation 9

| Ingredient | |
|---|---|
| Na soap from palm kernel oil | 2.0 |
| Sodium Citrate | 6.0 |
| MGDA | 1 |
| Alkyl benzene solfonates (LAS) | 3 |
| GLUCOLIPIDS | 15 |
| Phenoxyethanol | 0.45 |
| Medley^{®} Brilliant 300 L | 2 |
| | Add water to 100 % |
| pH | 8.2 |

### Example Formulation 10

| Ingredient | |
|---|---|
| Na soap from palm kernel oil | 2.0 |
| Sodium Citrate | 6.0 |
| MGDA | 1 |
| Sodium lauryl ether sulfate (SLES) | 5 |
| FAEO | 3 |
| GLUCOLIPIDS | 15 |
| Phenoxyethanol | 0.45 |
| Medley^{®} Brilliant 300 L | 2 |
| | Add water to 100 % |
| pH | 8.3 |

### Example Formulation 11

| Ingredient | | | |
|---|---|---|---|
| Na soap from palm kernel oil | 2 | 2 | 2 |
| Sodium Citrate | 6 | 6 | 6 |
| GLUCOLIPIDS | 15 | 15 | 15 |
| Trilon^{®} M | 1 | 1 | 1 |
| FAEO (C12-18 7EO) | - | 5 | - |
| Phenoxyethanol | 0.45 | 0.45 | 0.45 |
| NaOH (water solution) | 1,6 | 1,6 | 1,6 |
| Medley^{®} Brilliant 300 L | 3 | 2 | - |
| | Add water to 100 % | Add water to 100 % | Add water to 100 % |
| pH | 8.3 | 8.3 | 8.4 |

### Example Formulation 12-14

| Ingredient | | |
|---|---|---|
| Na soap from palm kernel oil | 2 | 2 |
| Sodium Citrate | 6 | 6 |
| GLUCOLIPIDS | 15 | 15 |
| Trilon^{®} M | 1 | 1 |
| TexCare^{®} SRN 260 Life | 1 | 2,5 |
| Phenoxyethanol | 0.45 | 0.45 |
| NaOH (water solution) | 1,6 | 1,6 |
| Medley^{®} Brilliant 300 L | 2 | 2 |
| | Add water to 100 % | Add water to 100 % |
| pH | 8.2 | 8.1 |

### Example Formulation 15-16

| Ingredient | | | |
|---|---|---|---|
| Na soap from palm kernel oil | 2 | 2 | 2 |
| Sodium Citrate | 6 | 6 | 6 |
| Rhamnolipids | - | 15 | 7.5 |
| GLUCOLIPIDS | 5 | 5 | 5 |
| Trilon^{®} M | 1 | 1 | 1 |
| Carboxy methyl inulin) Carboxyline^{®}CMI | 1 | 1 | 1 |
| Phenoxyethanol | 0.45 | 0.45 | 0.45 |
| NaOH (water solution) | 1,6 | 1,6 | 1,6 |
| Medley^{®} Brilliant 300 L | 2 | 2 | 2 |
| | Add water to 100 % | Add water to 100 % | Add water to 100 % |
| pH | 8.1 | 8.4 | 8.4 |

### Example Formulation 17-19

| Ingredient | | |
|---|---|---|
| Na soap from palm kernel oil | 2 | 2 |
| Sodium Citrate | 6 | 6 |
| GLUCOLIPIDS | 15 | 15 |
| Trilon^{®} M | 1 | 1 |
| Carboxy methyl inulin / Carboxyline^{®}CMI | 2.0 | 2.0 |
| Phenoxyethanol | 0.45 | 0.45 |
| NaOH (water solution) | 1,6 | 1,6 |
| Medley^{®} Brilliant 300 L | 2 | 2 |
| | Add water to 100 % | Add water to 100 % |
| pH | 8.1 | 8.4 |

### Example Formulation 20-21

| Ingredient | | |
|---|---|---|
| Na soap from palm kernel oil | 2 | 2 |
| Sodium Citrate | 6 | 6 |
| GLUCOLIPIDS | 15 | 15 |
| Trilon^{®} M | 1 | 1 |
| Carboxy methyl inulin ) Carboxyline^{®}CMI | | 1 |
| TexCare^{®} SRN 260 Life | 1 | |
| FAEO (C12-18 7EO) | 5 | 5 |
| Phenoxyethanol | 0.45 | 0.45 |
| NaOH (water solution) | 1,6 | 1,6 |
| Medley^{®} Brilliant 300 L | 2 | 2 |
| | Add water to 100 % | Add water to 100 % |
| pH | 8.1 | 8.1 |

### Example Formulation 22-23

| Ingredient | |
|---|---|
| Na soap from palm kernel oil | 2 |
| Sodium Citrate | 6 |
| GLUCOLIPIDS | 15 |
| Trilon^{®} M | 1 |
| TexCare^{®} SRN 170 | 1 |
| Phenoxyethanol | 0.45 |
| NaOH (water solution) | 1,6 |
| Medley^{®} Brilliant 300 L | 2 |
| | Add water to 100 % |
| pH | 8.4 |

### Example Formulation 24

| Ingredient | |
|---|---|
| Na soap from palm kernel oil | 2 |
| Sodium Citrate | 6 |
| GLUCOLIPIDS | 15 |
| Trilon^{®} M | 1 |
| Dehyton^{®} PL (lauramine oxide) | 1 |
| Phenoxyethanol | 0.45 |
| NaOH (water solution) | 1,6 |
| Medley^{®} Brilliant 300 L | 2 |
| | Add water to 100 % |
| pH | 8.4 |

### Example Formulation 25

| Ingredient | |
|---|---|
| Na soap from palm kernel oil | 2 |
| Sodium Citrate | 6 |
| GLUCOLIPIDS | 15 |
| Trilon^{®} M | 1 |
| REWOFERM SL ONE | 5 |
| Phenoxyethanol | 0.45 |
| NaOH (water solution) | 1,6 |
| Medley^{®} Brilliant 300 L | 2 |
| | Add water to 100 % |
| pH | 8.4 |

### Example Formulation 26

| Ingredient | |
|---|---|
| Na soap from palm kernel oil | 2 |
| Sodium Citrate | 6 |
| GLUCOLIPIDS | 15 |
| Trilon^{®} M | 1 |
| REWOFERM SL P | 5 |
| Phenoxyethanol | 0.45 |
| NaOH (water solution) | 1,6 |
| Medley^{®} Brilliant 300 L | 2 |
| | Add water to 100 % |
| pH | 8.4 |

### Example Formulation 27

| Ingredient | Bio pre-spotter 3 |
|---|---|
| Sodium Citrate | 6.0 |
| GLUCOLIPIDS | 10 |
| Monopropylene glycol | 2 |
| Phenoxyethanol | 0.45 |
| Protease | 1.2 |
| Amylase | 0.4 |
| Lipase | 0.4 |
| Pectinase | 0.2 |
| Carenzyme Premium | 0.15 |
| Celluclean 4500T | 0.15 |
| Mannanase | 0.2 |
| | Add water to 100 % |
| pH | 8.0 |

Example Formulation 28

## Claims

1. Composition comprising
A) at least one enzyme, and
B) at least one glucolipid of general formula (I) or a salt thereof, 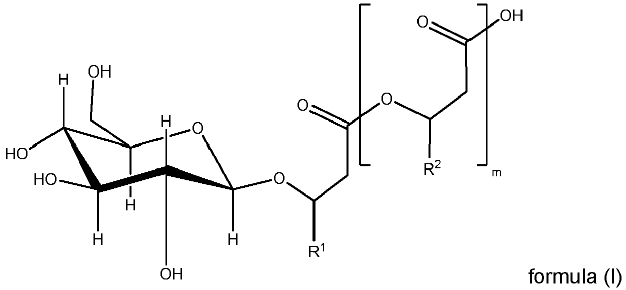
where
m = 1 or 0,
R¹ and R² = independently of one another identical or different organic radical having 2 to 24 carbon atoms, in particular optionally branched, optionally substituted, in particular hydroxy-substituted, optionally unsaturated, in particular optionally mono-, di- or triunsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12.

2. Composition according to claim1, **characterized in that** it comprises glucolipids of the general formula (I) or a salt thereof with m=1 and at least 51% by weight to preferably 98% by weight, preferably 60% by weight to 95% by weight, more preferably 70% by weight to 90% by weight, particularly preferably 75% by weight to 85% by weight, glucolipids GL-C10C10 of the general formula (I) with m=1 and R¹ and R² = (CH₂)₆-CH₃,where the percentages by weight refer to the sum of all of the glucolipids of the general formula (I) present.

3. Composition according to claim1 or 2, **characterized in that** the at least one enzyme is selected from the selected from the group consisting of protease, amylase, cellulase, mannanase, lipase, cutinase, pectate lyase, peroxidase, oxidase and laccase.

4. Composition according to at least one of the preceding claims, **characterized in that** it comprises
C) at least one builder.

5. Composition according to at least one of the preceding claims, **characterized in that** it comprises
A) in an amount of from 0.2 wt.-% to 10 wt.-%, preferably from 1.0 wt.-% to 8.0 wt.-%, more preferably from 1.5 wt.-% to 6.0 wt.-%, and as may be the case
B) in an amount of from 1.0 wt.-% to 60 wt.-%, preferably from 3.0 wt.-% to 40 wt.-%, more preferably from 6.0 wt.-% to 35 wt.-%, even more preferably from 10.0 wt.-% to 30 wt.-%, and as may be the case,
C) in an amount of from 1.0 wt.-% to 30 wt.-%, preferably from 2.0 wt.-% to 20 wt.-%, more preferably from 3.0 wt.-% to 10 wt.-%,
where the percentages by weight refer to the total composition.

6. Composition according to at least one of the preceding claims, **characterized in that** it comprises
at least one further surfactant, preferably selected from the group of non-glucolipid biosurfactants, anionic, cationic, non-ionic, semi-polar and zwitterionic surfactants.

7. Composition according to at least one of the preceding claims, **characterized in that** it is a detergent composition.

8. Use of a composition according to at least one of the Claims 1 to 7 for cleaning a surface of an article, preferably a textile or a fabric.

9. Use according to claim 8 **characterized in that** the surface of the article is cleaned from fat and/or oil.

10. Use of a glucolipid of general formula (I) or a salt thereof, 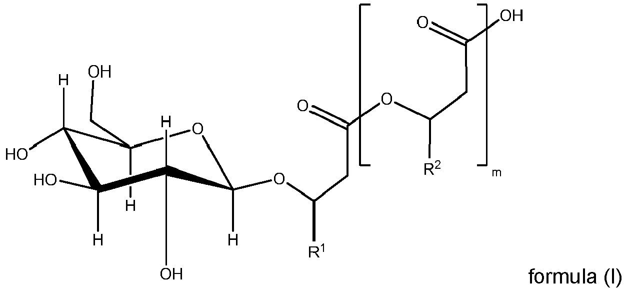 where
m = 1 or 0,
R¹ and R² = independently of one another identical or different organic radical having 2 to 24 carbon atoms, in particular optionally branched, optionally substituted, in particular hydroxy-substituted, optionally unsaturated, in particular optionally mono-, di- or triunsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12.
for the stabilization and/or elevation of an enzymatic activity of an enzyme, preferably selected from the group consisting of protease, amylase, cellulase, mannanase, lipase, cutinase, pectate lyase, peroxidase, oxidase and laccase with protease, amylase, lipase and mannanase being especially preferred and lipase being the most preferred.
